# EUROPÄISCHE PATENTSCHRIFT

(11) **EP 0 674 524 B1**
(45) Veröffentlichungstag und Bekanntmachung des Hinweises auf die Patenterteilung: **15.04.1998**
(21) Anmeldenummer: 94902772.6
(22) Anmeldetag: 15.12.1993
(51) Int. Cl.: A61K 38/00, A61K 9/14, A61K 47/18, A61K 47/26

(54) **STABILE LYOPHILISIERTE PHARMAZEUTISCHE ZUBEREITUNGEN VON G-CSF**
STABLE LYOPHILIZED PHARMACEUTICAL PREPARATIONS G-CSF
PREPARATIONS PHARMACEUTIQUES DE G-CSF LYOPHILISEES STABLES

(30) Priorität: 18.12.1992 DE 4242863
(43) Veröffentlichungstag der Anmeldung: 04.10.1995
(73) Patentinhaber: BOEHRINGER MANNHEIM GMBH, 68298 Mannheim (DE)
(72) Erfinder: MICHAELIS, Uwe, D-82362 Weilheim (DE); RUDOLPH, Rainer, D-82362 Weilheim (DE); WINTER, Gerhard, D-69221 Dossenheim (DE); WOOG, Heinrich, D-69514 Laudenbach (DE)
(86) Internationale Anmeldenummer: EP9303543
(87) Internationale Veröffentlichungsnummer: WO9414465

(56) Entgegenhaltungen:
- EP-A- 0 373 679
- EP-A- 0 528 313
- EP-A- 0 528 314
- WO-A-93/13752
- DE-A- 3 723 781

## Beschreibung

Gegenstand der vorliegenden Erfindung sind lyophilisierte pharmazeutische Zubereitungen von G-CSF, die Maltose, Raffinose, Saccharose, Trehalose oder Aminozucker als Stabilisierungsmittel enthalten. Außerdem betrifft die Erfindung ein Verfahren zur Herstellung dieser stabilisierten Lyophilisate sowie die Verwendung von Maltose, Raffinose, Saccharose, Trehalose oder Aminozucker als Stabilisatoren von G-CSF-haltigen Arzneimitteln.

Verschiedene pharmazeutische Zubereitungen, die G-CSF (Granulozyten-Kolonie stimulierender Faktor) enthalten, sind bereits aus dem Stand der Technik bekannt.

In DE 37 23 781 (GB 2,193,631) wird ein G-CSF-haltiges Arzneimittel beschrieben, das zur Stabilisierung von G-CSF mindestens ein pharmazeutisches grenzflächenaktives Mittel, Saccharid, Protein oder eine hochmolekulare Verbindung enthält. Es werden dort Zubereitungen vorgeschlagen, die Humanserumalbumin als stabilisierendes Mittel enthalten. Als vorteilhaft werden insbesondere Zubereitungen genannt, die oberflächenaktive Mittel in Gewichtsanteilen enthalten, die dem 1- bis 10 000fachen der eingesetzten G-CSF-Menge entsprechen.

In EP 0 373 679 werden stabilisierte Zubereitungen von GCSF beschrieben, die sich im wesentlichen durch einen sauren pHWert der Lösung auszeichnen, wobei die Lösungen eine möglichst geringe Leitfähigkeit aufweisen sollten. Die Lösungen besitzen einen pH-Wert von 3 - 3,7, falls die Lösungen weitere pharmazeutische Hilfsstoffe, wie beispielsweise Puffer oder Mannitol, enthalten. Für den Fall, daß keine Puffersubstanzen in der Arzneiform vorhanden sind, werden pH- Bereiche von 2,75 - 4 als vorteilhaft beschrieben.

Weiterhin werden in EP 0 306 824 stabilisierte Lyophilisate von Humanprotein-Präparaten beschrieben, bei denen die Stabilisierung durch Zusatz eines Gemisches von Harnstoff, Aminosäuren und Detergenz erfolgt.

In der früheren PCT-Patentanmeldung PCT/EP92/01823 wird ein Verfahren zur Herstellung von G-CSF-haltigen, gut verträglichen Arzneimitteln für Infusions- oder Injektionszwecke beschrieben. Die flüssigen Darreichungsformen zeichnen sich insbesondere durch eine geringe Titrationsacidität und geringe Pufferkapazität aus. Die pH-Werte der beschriebenen G-CSF- haltigen Infusions- und Injektionslösungen liegen im sauren Bereich von etwa 3,8 - 4,5.

Verfahren zur Herstellung von G-CSF-haltigen flüssigen Arzneiformen, die zusätzlich Konservierungsmittel enthalten, sind bekannt aus PCT/EP92/01822. Die pH-Werte der wässrigen pharmazeutischen Lösungen liegen im sauren Bereich von 2,5 - 4,5. Die Stabilisierung von G-CSF wird dort im wesentlichen durch die Einstellung des für G-CSF günstigen sauren pH-Wertes und durch Zugabe einer Mischung von verschiedenen Aminosäuren erreicht.

Die bisher bekannten Arzneiformen für G-CSF besitzen jedoch einige Nachteile. Es wurde festgestellt, daß flüssige GCSF-Zubereitungen gegenüber Einfrieren und Auftauen in einigen Fällen empfindlich sein können. Das unkontrollierte Einfrieren und Wiederauftauen derartiger Zubereitungen kann zur Entstehung von Dimeren, Oligomeren und Aggregaten führen, ggf. können auch unlösliche Präzipitate hervorgerufen werden. Derartige Eigenschaften von Proteinarzneimitteln sind aus medizinisch-pharmazeutischer Sicht bedenklich, da ein versehentliches Einfrieren der pharmazeutischen Lösung nicht mit Sicherheit vermieden werden kann, und somit das Risiko der Applikation einer qualitativ veränderten Zubereitung besteht.

Nachteilig bei den in DE 37 23 781 beschriebenen Zubereitungen ist ferner die Tatsache, daß diese pharmazeutische Zusatz- oder Hilfsstoffe enthalten, die aus medizinischer Sicht nicht ohne weiteres als unbedenklich einzustufen sind. Polymere und Proteine besitzen im Hinblick auf ihre Eignung als pharmazeutische Zusatzstoffe aufgrund ihrer Herkunft und physikalisch-chemischen Eigenschaften ein gewisses Risikopotential. Proteine menschlichen oder tierischen Ursprungs sowie aus Zellkulturen gewonnene Proteine tragen ein potentielles Restrisiko viraler Verunreinigungen. Auch andere, analytisch schwer nachweisbare proteinartige Verunreinigungen können wegen ihrer antigenen Eigenschaften immunologische Reaktionen beim Menschen hervorrufen. Proteine tierischen Ursprungs können darüber hinaus generell aufgrund ihrer spezies-spezifischen Eigenschaften immunologische Reaktionen beim Menschen auslösen. Auch Langzeitreaktionen nach späterer Reapplikation derartiger Proteine sind möglich.

Der Zusatz von hochmolekularen Verbindungen (Polymere) kann ebenfalls problematisch sein. Polymere sind aufgrund ihrer großen Molekülmasse im Körper akkumulierbar und können somit, falls kein Bioabbau erfolgt, über lange Zeit im Körper verbleiben. Dies ist besonders bei subkutaner Applikation zu befürchten, da der Abtransport und die Verteilung durch den Blutstrom gegenüber intravenöser Gabe stark verlangsamt erfolgt. In Abhängigkeit von der Molmasse können Polymere auch antigene Eigenschaften aufweisen. Auch ist die Reinheit von Polymeren aufgrund der zur Herstellung verwendeten Katalysatoren oder des Vorhandenseins von Monomeren und anderen Polymerbruchstücken schwierig zu gewährleisten. Der Einsatz von Polymeren bei pharmazeutischen Darreichungsformen, insbesondere bei subkutan applizierbaren Arzneiformen ist somit, wenn möglich, zu vermeiden.

Die in DE 37 23 781 beschriebenen Tensidmengen sind aus medizinischer Sicht ebenfalls als problematisch anzusehen. Dort werden Tensidkonzentrationen als vorteilhaft beschrieben, die bezüglich der Gewichtsanteile von G-CSF 1- bis 10 000 Gewichtsanteile eines oberflächenaktiven Mittels enthalten. Betrachtet man andererseits die für den klinischen Gebrauch bevorzugten Anwendungskonzentrationen von G-CSF von 0,05 - 1,5mg/ml in den fertigen Arzneiformen, so ergeben sich entsprechend hohe Tensidkonzentrationen. Diese sind aus medizinischer Sicht zu vermeiden, da sie lokale Irritationen auslösen können.

Außerdem haben einige der bekannten Formulierungen den Nachteil, daß sie aufgrund des angewandten niedrigen pH-Wertes insbesondere bei subkutaner Anwendung zu lokalen Unverträglichkeiten beim Patienten führen. Das erhaltene Produkt kann bei empfindlichen Patienten zu Schmerzen und lokaler Gewebereizung führen, da der im Gewebe physiologisch vorliegende Bereich von pH 7,0 - 7,5 nicht eingehalten wird.

Aus der Literatur ist ferner bekannt, daß insbesondere nichtglykosilierte Formen von G-CSF gegenüber glykosiliertem G-CSF, das aus CHO-Cellen gewonnen wird, besonders instabil sind (J. Biol. Chem. 1990, 265, 11432). Die Stabilisierung von nicht-glykosilierten Formen von G-CSF erwies sich als besonders schwierig und bedarf speziell ausgewählter Maßnahmen, um dieses Molekül in einer stabilen Arzneiform zu formulieren.

Aufgabe der vörliegenden Erfindung war es, eine Arzneiform für G-CSF zur Verfügung zu stellen, die eine ordnungsgemäße Anwendung von G-CSF als Arzneimittel ermöglicht und die oben beschriebenen Nachteile der bisher bekannten Arzneiformen nicht aufweist. Die pharmazeutische Zubereitung sollte sowohl stabil gegenüber unkontrollierten Einfrier- und Auftauvorgängen als auch stabil bei längerer Lagerung als Lyophilisat sein, physiologisch gut verträglich, einfach handhabbar und exakt dosierbar sein.

Die in DE 37 23 781 beschriebenen Beispiele zeigen, daß stabile Lyophilisate erhalten werden können, wenn Humanserumalbumin als Hilfsstoff eingesetzt wird. Der Zusatz von Zuckeralkoholen allein führt zu weniger stabilen Formulierungen. Es ist deshalb im Sinne einer Verbesserung des Standes der Technik wünschenswert, Formulierungen zu finden, die kein Humanserumalbumin (HSA) oder andere Proteine oder Polymere enthalten und dennoch gute Stabilität auch bei erhöhter Temperatur aufweisen. Der Verzicht auf Humanserumalbumin und Polymere vermindert das medizinische Risiko von Nebenwirkungen, wie sie beispielsweise für HSA beschrieben sind.

Überraschenderweise wurde gefunden, daß man im Sinne der vorliegenden Erfindung stabile pharmazeutische Arzneiformen herstellen kann, wenn man als Zusatzstoffe Maltose, Raffinose, Saccharose, Trehalose oder Aminozucker einsetzt.

Feste Zubereitungen, die Maltose, Raffinose, Saccharose, Trehalose oder Aminozucker als Hilfsstoffe enthalten, können ohne nennenswerten Qualitätsverlust des Proteins eingefroren oder auch bei erhöhten Temperaturen (bis 40 °C) gelagert werden. Die pharmazeutische Qualität des Wirkstoffes wird hierdurch nicht negativ beeinflußt. Die erfindungsgemäßen Zubereitungen werden vorzugsweise als Lyophilisate in den Handel gebracht. Die nach Redissolution hergestellten wässrigen Zubereitungen sind sehr gut verträglich, und stellen hinsichtlich der Proteinstabilität qualitativ hochwertige Zubereitungen dar. Sie haben außerdem den Vorteil, daß durch den Zusatz von Maltose, Raffinose, Saccharose, Trehalose oder Aminozuckern als Hilfsstoffe Lösungen mit einem vorteilhaften pH-Wert von 4 - 5 oder 7 - 8 hergestellt werden können, während die aus dem Stand der Technik bekannten Lösungen aus Stabilitätsgründen des Proteins hauptsächlich Lösungen mit einem pH- Wert von 2,5 - 3,5 erforderlich machten.

Die erfindungsgemäßen Zubereitungen besitzen außerdem den Vorteil, daß sie im wesentlichen frei von proteinartigen oder polymeren Hilfsstoffen sind, deren Verwendung aus medizinischer Sicht nicht unproblematisch sein kann. Aufgrund der Tatsache, daß nunmehr durch Auflösen von Lyophilisaten erhaltene, flüssige G-CSF-haltige Arzneiformen mit einem pH-Wert von etwa 4 - 5 oder 7 - 8, vorzugsweise mit einem pH-Wert in der Nähe des pH-Wertes des Blutes (pH 7,2 - 7,4), hergestellt werden können, besitzen sie ferner den Vorteil, gut verträglich und weitgehend schmerzfrei applizierbar zu sein. Dies ist vor allem bei subkutaner Gabe wesentlich, da hier leichter Unverträglichkeiten entstehen als bei intravenöser Gabe. Die erfindungsgemäßen Zubereitungen lassen sich auch in den klinisch besonders bevorzugten Konzentrationsbereichen von 0,05 - 1,5 mg/ml herstellen, so daß Injektionsvolumina von ≤ 1,0 ml eingehalten werden können. Kleine Injektionsvolumen sind bei subkutaner Applikation besonders vorteilhaft, da sie nur geringe mechanische Reize im Unterhautgewebe hervorrufen.

Vorteilhaft ist ferner, daß aufgrund der gewählten Hilfsstoffe die bisher benötigten relativ hohen Tensidmengen in den flüssigen Arzneiformen nicht mehr erforderlich sind. Vielmehr sind niedrige Tensidmengen von 0,5 mg/ml oder weniger, vorzugsweise von 0,01 - 0,1 mg/ml, ausreichend zur Stabilisierung von G-CSF. Vorteilhaft können Tensidkonzentrationen (mg/ml) verwendet werden, die kleiner als oder maximal gleich der eingesetzten Proteinmenge an G-CSF pro Volumeneinheit (mg/ml) sind. Dies ist vor allem bei solchen flüssigen Arzneiformen von Vorteil, die für die subkutane Anwendung von GCSF bestimmt sind. Außerdem werden durch die erfindungsgemäßen Maßnahmen insbesondere die labilen, unglykosilierten G-CSF-Moleküle für pharmazeutische Zubereitungen ausreichend stabilisiert.

Der Hilfsstoff Maltose (Malzzucker, Maltobiose, 4-O-alpha-D-Glucopyranosyl-D-glucose) wird in einer Menge der 0,01 - 10 000fachen Menge des Wirkstoffes G-CSF eingesetzt. Das Gleiche gilt für die Hilfsstoffe Raffinose, Saccharose und Trehalose. Die Konzentration dieser Hilfsstoffe in der flüssigen Arzneiform beträgt 0,1 - 200 mg/ml, vorzugsweise 10 - 60 mg/ml. Anstatt von Maltose können auch die stereoisomeren Disaccharide Cellobiose, Gentiobiose oder Isomaltose eingesetzt werden. Als Aminozucker werden generell solche Monosaccharide bezeichnet, die anstelle einer Hydroxygruppe eine Amino- oder eine acylierte Aminogruppe besitzen. Beispiele hierfür sind Glucosamin, Galactosamin, Neuraminsäure.

In einer besonderen Ausführungsform werden pharmazeutische Zubereitungen zur Verfügung gestellt, die neben Maltose, Raffinose, Saccharose oder Trehalose ferner Aminosäuren enthalten. Insbesondere kommen als Aminosäuren basische Aminosäuren in Frage, wie beispielsweise Arginin, Lysin, Ornithin, u.a., saure Aminosäuren, wie beispielsweise Glutaminsäure, Asparaginsäure, u.a. oder auch aromatische Aminosäuren, wie beispielsweise Phenylalanin, Tyrosin, Tryptophan, u.a. .

Aminosäuren werden in einer 0,01 - 10 000fachen Menge des Wirkstoffes G-CSF eingesetzt. Die Konzentration dieser Hilfsstoffe in der flüssigen Arzneiform beträgt 0,1 - 200 mg/ml, vorzugsweise 1 - 50 mg/ml.

Zur Herstellung der Lyophilisate werden zunächst die wässrigen pharmazeutischen Lösungen hergestellt, die den Wirkstoff und andere pharmazeutische übliche Hilfsstoffe enthalten. Als pharmazeutische Hilfsstoffe kommen insbesondere Aminosäuren, wie z. B. Arginin, Lysin, Ornithin, Phenylalanin oder Tyrosin in Frage. Außerdem kann die wässrige Zubereitung übliche Puffersubstanzen, wie z. B. Essigsäure, Salzsäure, Zitronensäure, Milchsäure, Weinsäure, Maleinsäure und Phosphorsäure oder deren physiologisch verträglichen Salze enthalten. Bei der Herstellung der Hilfsstofflösung können diese Puffersubstanzen entweder in Form der entsprechenden freien Säure oder in Form der Alkali-, Erdalkali- oder Ammoniumsalze vorgegeben werden. Außerdem kann die Lösung weitere pharmazeutisch übliche Hilfsstoffe bereits enthalten.

Die Reihenfolge der Zugabe der verschiedenen Hilfsstoffe oder des Wirkstoffes ist weitgehend unabhängig hinsichtlich des Herstellungsverfahrens und liegt im Ermessen des Fachmannes. Der gewünschte pH-Wert der Lösung wird durch Zugabe von Basen, wie beispielsweise von Alkalihydroxiden, Erdalkalihydroxiden oder Ammoniumhydroxid eingestellt. Vorzugsweise wird hierzu Natriumhydroxid verwendet. Die Einstellung des gewünschten pH-Wertes ist prinzipiell durch Zugabe von basischen Lösungen möglich. In diesem Sinne kommen allgemein Salze von starken Basen mit schwachen Säuren in Frage, wie z. B. Natriumacetat, Natriumcitrat, Di-Natrium- bzw. Di-Kaliumhydrogenphosphat oder Natriumcarbonat. Für den Fall, daß die pharmazeutische Hilfsstofflösung einen basischen pHWert aufweist, erfolgt die Einstellung durch Titration mit Säure, bis der gewünschte pH-Bereich erreicht ist. Als Säuren kommen physiologisch verträgliche anorganische oder organische Säuren in Frage, wie beispielsweise Salzsäure, Phosphorsäure, Essigsäure, Zitronensäure oder allgemein übliche Lösungen von Substanzen, die einen sauren pHWert besitzen. Bevorzugte Substanzen sind in diesem Sinne Salze von starken Säuren mit schwachen Basen, wie z. B. Natriumdihydrogenphosphat oder Kaliumdihydrogenphosphat.

Die Konzentrationen der Puffersubstanzen in der fertig applizierbaren flüssigen Arzneiform betragen jeweils etwa 2 - 80mMol/l. Die Gesamtkonzentration an Puffersubstanzen sollte einen Wert von 100mMol/l nicht übersteigen. Bevorzugt beträgt die Konzentration der Puffersubstanzen 5 - 40 mMol/l.

Die mittels der genannten Hilfsstoffe erfolgte Stabilisierung von G-CSF-Molekülen bezieht sich prinzipiell auf alle durch rekombinante Verfahren hergestellte G-CSF-Moleküle und deren Varianten. Der Begriff G-CSF oder G-CSF-Variante gemäß vorliegender Erfindung beinhaltet alle natürlich vorkommenden Varianten von G-CSF, sowie davon abgeleitete durch rekombinante DNA-Technologie modifizierten GCSF-Proteine, insbesondere Fusionsproteine, die neben dem GCSF-Anteil noch andere Proteinsequenzen enthalten. Besonders bevorzugt ist in diesem Sinne G-CSF-Mutein mit einem N-terminalen Met-Rest an Position - 1, das zur Expression in prokaryontischen Zellen geeignet ist. Ebenso geeignet ist eine rekombinante Methionin-freie G-CSF-Variante, die gemäß PCT/EP91/00192 hergestellt werden kann. Unter dem Begriff "GCSF-Variante" werden solche GCSF-Moleküle verstanden, bei denen eine oder mehrere Aminosäuren deletiert oder durch andere Aminosäuren ersetzt sein können, wobei die wesentlichen Eigenschaften von G-CSF weitgehend erhalten bleiben. Geeignete G-CSF-Muteine sind beispielsweise in EP0 456 200 beschrieben.

Zur Herstellung gut verträglicher parenteraler Arzneiformen ist der Zusatz von isotonisierenden Hilfsstoffen zweckmäßig, wenn nicht durch die osmotischen Eigenschaften des Wirkstoffes und der zur Stabilisierung eingesetzten Hilfsstoffe bereits Isotonie erreicht werden kann. Dazu werden vor allem nicht-ionisierte, gut verträgliche Hilfsstoffe eingesetzt.

Der Zusatz von Salzen ist zur Einstellung der Isotonie nicht vorteilhaft, da hohe Salz- oder Ionenkonzentrationen die Aggregatbildung von G-CSF fördern. Vorteilhaft werden deshalb Salze in geringer Menge zugesetzt.

Außerdem können die pharmazeutischen Zubereitungen weitere übliche Hilfs- oder Zusatzstoffe enthalten. Es können Antioxidanzien, wie beispielsweise Glutathion oder Ascorbinsäure oder ähnliche Substanzen, chaotrope Hilfsstoffe, wie beispielsweise Harnstoff, und Aminosäuren, wie beispielsweise Arginin, Lysin, Ornithin, Glutaminsäure und andere zugesetzt werden.

Im folgenden wird die Erfindung anhand von repräsentativen Ausführungsbeispielen näher beschrieben:

Die Beispiele 1 - 14 zeigen, in welcher Weise erfindungsgemäße Lyophilisate formuliert, hergestellt und hinsichtlich der Stabilität des Proteins näher untersucht werden können. Der Einfluß der neben Maltose, Raffinose, Saccharose oder Trehalose zugesetzten Hilfsstoffe sowie des pH-Wertes wird erläutert.

Vergleichende Untersuchungen zu auf der Basis von Mannit oder Glycin hergestellten Lyophilisaten zeigen, daß Maltose-, Raffinose-, Saccharose- oder Trehalose-Lyophilisate signifikant bessere Ergebnisse liefern, als die mit anderen Gerüstbildnern hergestellten Zubereitungen. Durch Einsatz der erfindungsgemäß beschriebenen und in den Beispielen erläuterten Lyophilisate läßt sich im Sinne der beschriebenen Zielsetzung eine optimale Formulierung herstellen, die einen physiologisch verträglichen pH-Wert aufweist, langfristig lagerstabil ist und dabei erhöhte Lagertemperaturen sowie mechanischen Streß ohne negative Auswirkungen auf das Protein aushält. Die Zubereitungen sind insbesondere gegen Einfrieren unempfindlich und auf die als kritisch angesehenen Hilfsstoffe, wie beispielsweise Proteine oder Polymere, kann völlig verzichtet werden. Außerdem sind nur relativ geringe Mengen an physiologisch gut verträglichen Tensiden enthalten.

In Beispiel 3 werden verschiedene Zucker oder Zuckeralkohole auf ihre stabilisierende Wirkung in G-CSF-Lyophilisaten untersucht. Maltose zeigt sich gegenüber Lactose und Mannit vorteilhaft.

In Beispiel 4 werden Lyophilisate mit Maltose und weiteren Hilfsstoffen beschrieben. Die Ergebnisse belegen klar, daß der Zusatz von Tensid die Stabilität der Zubereitung nicht wesentlich beeinflußt, jedoch die Anhaftung des Proteins an Oberflächen und damit mögliche Gehaltsverluste verhindert. Die Anwesenheit von Tensid ist somit in derartigen Rezepturen nicht aus Gründen der Stabilisierung, sondern zur Aufrechterhaltung der Nenndosierung erforderlich.

In Beispiel 5 werden verschiedene Maltose-haltige Lyophilisat-Rezepturen verglichen mit zwei ansonsten gleichartig formulierten Lyophilisaten ohne Maltose. Aus den Daten ist klar erkennbar, daß die Anwesenheit von Maltose in den untersuchten Parametern vorteilhaft im Sinne der Stabilität der Zubereitung wirkt. Der Zusatz weiterer Hilfsstoffe, wie Ascorbinsäure, Glutathion, Glutaminsäure zeigt im Rahmen der untersuchten Lagertemperaturen und Lagerzeiten keinen signifikanten Einfluß auf die Stabilität. Die in Beispiel 5 beschriebenen Zubereitungen zeichnen sich insbesondere dadurch aus, daß sie bei langfristiger Lagerung bei erhöhter Temperatur keine Veränderungen in den untersuchten Qualitätsmerkmalen aufweisen.

Aus den Beispielen ist weiterhin ersichtlich, daß in Lyophilisaten, die Maltose und Arginin enthalten, ein weiteres Puffersalz nicht unbedingt erforderlich ist, da der bei pHEinstellung durch Salzsäure, Phosphorsäure, Zitronensäure oder anderen Säuren entstehende Argininpuffer ausreichend pH-stabilisierend wirkt. Argininpuffer ist hervorragend geeignet, stabile Zubereitungen im pH-Bereich unter 5,0 und von 7,0 - 7,5 zu formulieren (siehe Beispiele 11 und 12). Beispiel 9 zeigt, daß wiederaufgelöste Lyophilisate mit pH 7,4, die Maltose und Argininpuffer enthalten, mindestens 24 Stunden haltbar sind.

In Beispiel 6 werden G-CSF-Lyophilisate beschrieben, die Aminozucker (Galactosamin, N-Methylglucosamin) enthalten. Es ist erkennbar, daß die Kombination von Maltose und Aminozucker stabilere Zubereitungen ergibt als die Kombination von Glycin mit Aminozuckern. Damit wird belegt, daß Maltose in Kombination mit physiologisch gut verträglichen Hilfsstoffen deutlich stabilere und damit bezüglich der pharmazeutischen Qualität hochwertigere Lyophilisate von G-CSF liefert als andere, in der Literatur vorgeschlagene Gerüstbildner und Stabilisatoren.

In Beispiel 7 wird gezeigt, daß G-CSF in Maltose-haltigen Lyophilisaten deutlich stabiler als in Mannit-haltigen Lyophilisaten ist. Dies wird bei relevanten Lagertemperaturen und langen Lagerzeiten entsprechend belegt.

In Beispiel 8 wird gezeigt, daß Maltose-haltige Lyophilisate bei verschiedenen pH-Werten sowie bei verschiedenen Hilfsstoffzusätzen vorteilhafte Ergebnisse gegenüber Lyophilisaten mit anderen Gerüstbildnern und Stabilisatoren (Zuckeralkoholen, Aminosäuren) erbringen.

Beispiel 10 belegt die Stabilität der erfindungsgemäßen Lyophilisate mit Maltose, Raffinose, Saccharose oder Trehalose nach 13-wöchiger Lagerung bei 40 °C.

Beispiel 11 zeigt, daß die erfindungsgemäßen Lyophilisate auch mit höheren G-CSF-Konzentrationen stabil sind, und in Beispiel 12 wird die Langzeitstabilität der erfindungsgemäßen Rezepturen selbst bei höheren Temperaturen belegt.

### Beispiel 1:

### Untersuchungsmethoden zur Stabilitätsbestimmung

Die lyophilisierten Zubereitungen wurden unter Lichtausschluß bei definierten Lagertemperaturen gelagert und danach mit reversed phase HPLC (RP-HPLC), Gelchromatographie oder size exclusion Chromatographie (SEC HPLC), Western Blot auf Proteinreinheit sowie das Auftreten von Aggregaten und Dimeren hin untersucht. Außerdem wurde der Proteingehalt durch OD 280-Photometrie, die Biologische Aktivität durch Bioassay (NFS 60-Zelltest), sowie Aggregation und Präzipitation durch Trübungsmessung untersucht. Die angewandten Methoden lassen sich wie folgt beschreiben:

### 1.1 Reversed phase HPLC

Die RP-HPLC erfolgte unter Verwendung einer Nucleosil C18- Säule (Fa. Knauer). Die mobile Phase bestand aus 0,12 % (v/v) Trifluoressigsäure (TFA)/Wasser (A) und 0,1 % (v/v) TFA/Ace- tonitril (B). Die Chromatographie wurde mit einer Flußrate von 0,5 ml/min unter Verwendung eines linearen Gradienten von A nach B durchgeführt.

Die Injektionsmenge betrug je nach Rezeptur 3 - 6 µg G-CSF. Die Auswertung erfolgte über die Peakfläche unter Verwendung eines externen Standards bei einer Wellenlänge von 214nm.

### 1.2 Size Exclusion Chromatographie (SEC)

Für die SE-Chromatographie wurde eine TSK G 2000 SW-Trennsäule (7,5 x 300 mm) verwendet. Die Trennungen erfolgten isokratisch bei Raumtemperatur und einer Flußrate von 0,6ml/min in einem Phosphatpuffer (22,2 mM Na₂HP04; 107,7mM KH₂PO₄; pH 6,2). Die Injektionsmenge betrug 3 - 6 µg G-CSF. Die Auswertung erfolgte über die Peakfläche unter verwendung eines externen Standards bei einer Detektionswellenlänge von 214nm.

### 1.3 SDS-Page/Western Blot

3 µg rhG-CSF werden unter nichtreduzierenden Bedingungen auf ein 12prozentiges Polyacrylamid-SDS-Gel gegeben und der Gelelektrophorese unterzogen. Anschließend werden die nach ihrem Molekulargewicht getrennten G-CSF-Monomere, -Dimere oder -Aggregate durch Elektroblotting auf Nitrocellulose transferiert. Durch Inkubation mit einem spezifischen polyklonalen biotinylierten Anti-G-CSF-Antikörper (PAK < GCSF >IgG) werden die Proteinbanden identifiziert und mittels der Phosphatase-Technik unter Benutzung von Streptavidin-alkalischem Phosphatasekonjugat (SA-AP-Konjugat), 5-Brom-4-chlor-3-indolylphosphat (BCIP) und Nitro Blue Tetrazolin (NBT) nachgewiesen. Die Bestimmung der prozentualen Monomeren-, Dimeren- bzw. Aggregatanteile erfolgt durch laserdensitometrische Auswertung mit Hilfe einer rhG-CSF-Standardreihe.

### 1.4 NFS-60 Bioassay (biologische Wirksamkeit)

Die in vitro Aktivitätsbestimmung von G-CSF basiert auf der Messung von Zellzahlen in einer durch G-CSF stimulierten Zellkultur von NFS-60 Zellen.

Unter geeigneten Bedingungen kann die Dehydrogenase-Aktivität der Zellen mit der Konzentration an G-CSF im Medium korreliert werden. Es werden geeignete Verdünnungen der G-CSF Pufferlösung hergestellt, um einen einfach meßbaren Anstieg in der Dehydrogenase-Aktivität zu erhalten.

Die Messung der Aktivität erfolgt dann photometrisch bei 570 und 690 nm, gemessen wird die Reduktion des Tetrazolinium-Salzes MTT (gelb) zu Formazan (blau).

Die in vitro Aktivität von G-CSF wird berechnet, indem die Daten der Probe gegen Standard nach der Methode der parallelen Linie verglichen werden. Die Auswertung erfolgt gemäß den Anforderungen der Ph. Eur. (VIII, 13).

### 1.5 Streulichtmessung, Trübungsbestimmung

Die Messung erfolgt direkt an der unverdünnten Produktlösung in Glasküvetten (Durchmesser 2 cm). Das von der Flüssigkeit diffus abgelenkte Streulicht wird unter einem Winkel von 90 °C gemessen. Gemessen wird im Vergleich zu Formazin-Standard-Suspensionen nach DIN 38404C2, die Angabe der Werte erfolgt in TE/F. Die Messung erfolgt an einem geeigneten Trübungsphotometer, z. B. LTP 5 (Fa. Dr. Lange, Düsseldorf).

### 1.6 Photometrie OD 280 (Proteingehalt)

Das G-CSF-UV-Spektrum hat ein Absorptionsmaximum bei 280 nm, das auf Seitenkettenchromophore wie Tryptophan-, Tyrosin- und Phenylalaninreste zurückzuführen ist. Die Messung erfolgt im Vergleich zu Placebo-Lösungen mittels:
- UV-Spectrophotometer
   (z. B. Uvikon 810 P oder 941, Kontron Instruments)
- Semi-micro Quartz Küvetten, 500 µl, Schichtdicke: 1 cm
   (z. B. Hellma, Suprasil, Cat. No. 104.002B-QS)

### Beispiel 2:

Wässrige Lösungen von 0,1 mg/1 ml Poloxamer 118 sowie 50mg/ml der nachfolgend genannten Zucker bzw. Zuckeralkohole Mannit (Rezeptur 1), Lactose (Rezeptur 2) und Maltose (Rezeptur 3) wurden mit G-CSF in einer Konzentration von 70 µg/ml versetzt. Die Lösungen wurden nach Filtration durch einen sterilisierten 0,2 µm-Membranfilter in sterile Injektionsflaschen aus Glas der hydrolytischen Klasse I abgefüllt. Nach der Lyophilisation wurde mit sterilem Stickstoff belüftet und die zunächst lose aufgesetzten Stopfen wurden zum Verschließen der Lyophilisate unter aseptischen Bedingungen eingedrückt. Die Lyophilisate wurden verbördelt und unter Lichtausschluß 6 und 13 Wochen bei verschiedenen Temperaturen gelagert. Danach wurde mit den nachfolgend beschriebenen Methoden die Stabilität der Zubereitung untersucht.

**Tabelle 1a**

| Lagerung bei 20 °C | | | | | | |
|---|---|---|---|---|---|---|
| | **Lagerung 6 Wochen bei 20 °C** | | | **Lagerung 13 Wochen bei 20 °C** | | |
| | **I** | **II** | **III** | **I** | **II** | **III** |
| | | | | | | |
| **Rez. 1 Mannit** | > 99 % | 91 % | 36 % | 86 % | 47 % | 27 % |
| **Rez. 2 Lactose** | > 99 % | > 99 % | 18 % | > 99 % | > 99 % | 12 % |
| **Rez. 3 Maltose** | > 99 % | > 99 % | 6 % | > 99 % | > 99 % | 7,8 % |
| I Reinheit unverändertes Protein in RP HPLC II Reinheit unverändertes Protein in SEC HPLC III Dimeren/Aggregate in Western Blot | | | | | | |

**Tabelle 1b**

| Lagerung bei 40 °C | | | | | | |
|---|---|---|---|---|---|---|
| | **Lagerung 6 Wochen bei 40 °C** | | | **Lagerung 13 Wochen bei 40 °C** | | |
| | **I** | **II** | **III** | **I** | **II** | **III** |
| | | | | | | |
| **Rez. 1 Mannit** | 81 % | 70 % | 50 % | 69 % | 25 % | 70 % |
| **Rez. 2 Lactose** | > 99 % | > 99 % | 37 % | > 99 % | > 99 % | nicht auswertbar/aggregiert |
| **Rez. 3 Maltose** | > 99 % | > 99 % | 6,4 % | > 99 % | > 99 % | 12 % |
| I Reinheit unverändertes Protein in RP HPLC II Reinheit unverändertes Protein in SEC HPLC III Dimeren/Aggregate in Western Blot | | | | | | |

### Beispiel 3:

Es wurden Lyophilisate von GCSF hergestellt. Dazu wurden die in nachfolgender Tabelle genannten Hilfsstoffe in Wasser für Injektionszwecke gelöst, danach wurde G-CSF in einer Konzentration von 70 µg/ml zugefügt und ggf. wurde mit geringen Mengen des Puffersystems der pHWert genau eingestellt. Pluronic F 68 wurde als ein Vertreter eines entsprechend geeigneten Tensides verwendet. Andere Tenside verhalten sich ähnlich. Nach Sterilfiltrationen durch einen geeigneten 0,2 µm-Membranfilter wurden die Lösungen in sterile Injektionsflaschen aus Glas der hydrolytischen Klasse I abgefüllt und nach üblichen Verfahren lyophilisiert. Nach der Lyophilisation wurde mit Stickstoff belüftet und die Injektionsflaschen mit Gefriertrocknungsstopfen unter aseptischen Bedingungen verschlossen. Die Zubereitungen wurden in verbördelten Flaschen unter Lichtausschluß bei definierten Lagertemperaturen bei 6 und 12 Wochen gelagert und mit den in Beispiel 1 genannten Methoden untersucht.

**Tabelle 2**

| Maltose-haltige Rezepturen von G-CSF bei pH 3,6 | | |
|---|---|---|
| | **Rezeptur 4** | **Rezeptur 5** |
| **G-CSF** | 70 µg | 70 µg |
| **Maltose** | 35 mg | 35 mg |
| **L-Phenylalanin** | 10 mg | 10 mg |
| **Ascorbinsäure** | 5 mg | 5 mg |
| **Glutathion** | 10 mg | 10 mg |
| **L-Glutaminsäure** | 5 mg | 5 mg |
| **L-Arginin** | 10 mg | 10 mg |
| **Puffer (pH)** | ad pH 3,6 | ad pH 3,6 |
| **Pluronic F 68** | - | 0,1 mg |
| **Wasser für Injektionszwecke** | ad 1 ml | ad 1 ml |

**Tabelle 3**

| Lagerung bei 20 °C | | | | | | | |
|---|---|---|---|---|---|---|---|
| **Rp.** | **Lager- temp.** | **nach 6 Wochen Aggr. in %** | **nach 12 Woche Aggr. in %** | **nach 6 Wochen** | | **nach 12 Wochen** | |
| | | | | **SEC % G-CSF** | **RP % G-CSF** | **SEC % G-CSF** | **HPLC % G-CS** |
| | | | | | | | |
| **4** | + 20 °C | 0,0 | 2,6 | 61 | 63 | 64 | 69 |
| **5** | + 20 °C | 0,0 | 0,5 | > 99 | > 99 | > 99 | > 99 |

### Beispiel 4:

G-CSF-Lyophilisate mit 500 µg/ml G-CSF (Rezepturen 6 - 10) wurden wie folgt hergestellt. Die in nachfolgender Tabelle genannten Hilfsstoffe wurden in Wasser für Injektionszwecke gelöst, GCSF wurde zugegeben und, falls nötig, wurde der pHWert mit geringen Mengen Salzsäure oder oder Di-Natrium-hydrogenphosphat einjustiert. Jeweils 1 ml der zuvor durch ein 0,2 µm-Membranfilter sterilfiltrierten Lösungen wurde in Injektionsflaschen aus Glas der hydrolytischen Klasse I abgefüllt und nach üblichen Verfahren gefriergetrocknet. Nach der Lyophilisation wurde mit Stickstoff belüftet und die Lyophilisate wurden mit Gefriertrocknungsstopfen unter aseptischen Bedingungen verschlossen. Die verbördelten Lyophilisate wurden unter Lichtausschluß bei definierten Temperaturen gelagert und mit den in Beispiel 1 genannten Methoden untersucht.

**Tabelle 4**

| Zusammensetzungen der Rezepturen 6 - 10 | | | | | |
|---|---|---|---|---|---|
| | **Rez. 6** | **Rez. 7** | **Rez. 8** | **Rez. 9** | **Rez. 10** |
| | | | | | |
| **G-CSF** | 0,5 µg | 0,5 µg | 0,5 µ | 0,5 µg | 0,5 µg |
| **Maltose** | 35 mg | 35 mg | 35 mg | - | - |
| **L-Phenylalanin** | 10 mg | 10 mg | 10 mg | 10 mg | 10 mg |
| **Ascorbinsäure** | 5 mg | - | - | 5 mg | - |
| **Glutathion** | 10 mg | - | - | 10 mg | - |
| **L-Glutaminsäure** | 5 mg | - | - | 5 mg | - |
| **L-Arginin** | 10 mg | 10 mg | 10 mg | 10 mg | 10 mg |
| **Puffer (pH)** | ad 4,5 | ad 4,5 | ad 6,5 | ad 4,5 | ad 6,5 |
| **Pluronic F68** | 0,1 mg | 0,1 mg | 0,1 mg | 0,1 mg | 0,1 mg |
| **Wasser für Injektionszw.** | ad 1 ml | ad 1 ml | ad 1 ml | ad 1 ml | ad 1 ml |

**Tabelle 5**

| Analysenergebnisse | | | | | | | | | |
|---|---|---|---|---|---|---|---|---|---|
| **Rez.** | **Lager- temp.** | **Western Blot** | | **nach 6 Wochen** | | | **nach 13 Wochen** | | |
| | | **6 Wo. % Aggr.** | **12 Wo. % Aggr.** | **SEC % G-CSF** | **% Aggr.** | **RP % G-CSF** | **SEC % G-CSF** | **% Aggr.** | **RP % G-CSF** |
| | | | | | | | | | |
| **6** | + 8 °C | < 1 | 1,0 | > 99 % | 0,9 | > 99 % | > 99 % | 0,7 | 99 % |
| | + 40 °C | < 1 | 1,7 | > 99 % | 0,6 | > 99 % | > 99 % | 0,6 | 98 % |
| | | | | | | | | | |
| **7** | + 8 °C | < 1 | 1,1 | > 99 % | 1,6 | > 99 % | > 99 % | 1,1 | 99 % |
| | + 40 °C | < 1 | 2,3 | > 99 % | 1,9 | > 99 % | > 99 % | 1,1 | 99 % |
| | | | | | | | | | |
| **8** | + 8 °C | < 1 | - | | | | > 98 % | 1,5 | > 99 % |
| | + 40 °C | < 1 | - | | | | > 98 % | 1,4 | > 99 % |
| | | | | | | | | | |
| **9** | + 8 °C | 3,8 | 0,3 | 95 % | 5,8 | > 99 % | 95 % | 1,5 | 98 % |
| | + 40 °C | 7,9 | 2,5 | 95 % | 6,4 | 93 % | 86 % | 0,8 | 94 % |
| | | | | | | | | | |
| **10** | + 8 °C | - | 5,2 | | | | 96 % | 1,0 | 95 % |
| | + 40 °C | - | 10,3 | | | | 89 % | 2,6 | 89 % |

### Beispiel 5:

Die in der nachfolgenden Tabelle angegebenen Formulierungen (Rezepturen 11 - 14) wurden wie folgt hergestellt: Die Hilfsstoffe wurden in Wasser für Injektionszwecke gelöst, danach G- CSF in der angegebenen Konzentration zugefügt. Falls notwendig, wurde der pH mit Hilfe der Komponenten des Phosphatpuffers genau einjustiert. Die Lösungen wurden sodann durch einen sterilisierten Membranfilter der Porenweite 0,2 µm filtriert und unter aseptischen Bedingungen in Injektionsflaschen der hydrolytischen Klasse I abgefüllt und lyophilisiert. Nach der Lyophilisation wurde mit Stickstoff belüftet, die Lyophilisate wurden mit Gefriertrocknungsgummistopfen unter aseptischen Bedingungen verschlossen und verbördelt. Die Lyophilisate wurden unter Lichtausschluß bei definierten Lagertemperaturen belastet. Nach 6 und 13 Wochen wurden Untersuchungen gemäß der in Beispiel 1 angegebenen Methoden durchgeführt.

**Tabelle 6**

| Aminozuckerhaltige Lyophilisatzubereitungen | | | | |
|---|---|---|---|---|
| | **Rez. 11** | **Rez. 12** | **Rez. 13** | **Rez. 14** |
| | | | | |
| **G-CSF** | 0,5 µg | 0,5 µg | 0,5 µg | 0,5 µg |
| **Pluronic F68** | 0,1 mg | 0,1 mg | 0,1 mg | 0,1 mg |
| **N-methylglucosamin** | - | 10 mg | - | 10 mg |
| **Galactosamin** | 10 mg | - | 10 mg | - |
| **Glycin** | **-** | **-** | 35 mg | 35 mg |
| **Maltose** | 35 mg | 35 mg | - | - |
| **Phenylalanin** | 10 mg | - | - | - |
| **Phosphatpuffer** | ad pH 7,0 | ad pH 7,0 | ad pH 7,0 | ad pH 7,0 |
| **Wasser für Injektionszwecke** | ad 1,0 ml | ad 1,0 ml | ad 1,0 ml | ad 1,0 ml |

Die nach Lagerung der o. g. Zubereitung erhaltenen Analysendaten sind in nachfolgender Ergebnistabelle zusammengefaßt.

**Tabelle 7**

| Analysenergebnisse ZSP = Zersetzungsprodukte | | | | | |
|---|---|---|---|---|---|
| **Rez.** | **Lagerung** | **6 Wochen West. Blot. % Aggr.** | **12 Wochen West. Blot. % Aggr.** | **12 Wochen** | |
| | | | | **RP-HPLC % G-CSF** | **% ZSP in SEC HPLC** |
| | | | | | |
| **11** | + 8 °C | 3,8 | 2,9 | > 99 | 1,2 |
| | + 40 °C | 3,2 | 2,3 | > 99 | 1,8 |
| | | | | | |
| **12** | + 8 °C | 1,8 | 3,8 | > 99 | 1,4 |
| | + 40 °C | 1,7 | 4,5 | > 99 | 0,7 |
| | | | | | |
| **13** | + 8 °C | 1,1 | 1,4 | > 99 | 0,9 |
| | + 40 °C | 16,8 | 13,0 | 75 | 4,2 |
| | | | | | |
| **14** | + 8 °C | 1,6 | 12,4 | 97,5 | 1,2 |
| | + 40 °C | 7,7 | 26,3 | 84,5 | 3,5 |

### Beispiel 6:

Die nachfolgend beschriebenen Rezepturen 15 und 16 wurden wie folgt hergestellt: Die angegebenen Hilfsstoffe wurden in Wasser für Injektionszwecke gelöst, G-CSF in der angegebenen Konzentration wurde zugefügt. Der pH wurde, falls notwendig, mit Anteilen der Pufferkomponenten einjustiert. Danach wurde die Lösung durch einen sterilisierten Membranfilter der Porenweite 0,2 µm filtriert und unter aseptischen Bedingungen in sterile Injektionsflaschen aus Glas der hydrolytischen Klasse I abgefüllt. Nachfolgend wurden die Injektionszubereitungen gefriergetrocknet, danach wurde mit Stickstoff belüftet und die Injektionsflaschen wurden unter aseptischen Bedingungen mit einem Gefriertrocknungsstopfen verschlossen und danach verbördelt. Die Zubereitungen wurden unter Lichtausschluß bei definierten Temperaturen gelagert und auf die nachfolgend genannten Parameter hin untersucht. Dabei wurden die in Beispiel 1 beschriebenen Untersuchungsmethoden angewandt.

| Rezeptur 15 | | Rezeptur 16 | |
|---|---|---|---|
| **G-CSF** | **0,25 mg** | **G-CSF** | **0,3 mg** |
| **Polysorbat 80** | 0,05 mg | Polysorbat 80 | 0,1 mg |
| **Phenylalanin** | 5 mg | Mannit | 50 mg |
| **Maltose** | 17,5 mg | Puffer | ad pH 4,5 |
| **L-Arginin** | 5 mg | Wasser f. Injektionszw. | ad 1,0 ml |
| **Puffer** | ad pH 4,5 | | |
| **Wasser f. Injektionszw.** | ad 0,5 ml | | |

**Tabelle 8**

| Untersuchungsergebnisse nach Lagerung von Rezeptur 15 und 16 über 3 und 6 Monate | | | | | | | | |
|---|---|---|---|---|---|---|---|---|
| | **Rezeptur 15** | | | | **Rezeptur 16** | | | |
| | **Lagerzeit 3 Monate** | | **Lagerzeit 6 Monate** | | **Lagerzeit 3 Monate** | | **Lagerzeit 6 Monate** | |
| | **4-8 °C** | **23 °C** | **4-8 °C** | **23 °** | **4-8 °C** | **23 °C** | **4-8 °C** | **23 °** |
| | | | | | | | | |
| **West. Blot (Dimere)** | 2,2 | < 1 % | 1,3 % | 0,7 | < 1 % | 12 % | 4,1 % | 17 |
| **SEC-HPLC (Dimere)** | < 1 % | < 1 % | < 1 % | < 1 % | < 1 % | 2 % | < 1 % | 3 |
| **RP-HPLC (G-CSF Peak)** | > 99 % | > 99 % | > 98 % | > 98 | > 99 % | 98,2 % | > 98 % | > 98 |

### Beispiel 7:

Die in Tabelle 9 beschriebenen Formulierungen wurden wie folgt hergestellt:

Die angegebenen Hilfsstoffe wurden in Wasser für Injektionszwecke gelöst, G-CSF wurde in der angegebenen Konzentration zugegeben, danach wurde, falls notwendig, der pHWert mit kleinen Anteilen der Pufferkomponenten einjustiert. Die Arzneistofflösung wurde dann durch einen sterilisierten Membranfilter der Porenweite 0,2 µm sterilfiltriert, danach unter aseptischen Bedingungen in sterilisierte Injektionsflaschen aus Glas der hydrolytischen Klassel abgefüllt und lyophilisiert.

Nach der Lyophilisation wurde mit Stickstoff belüftet und die Flaschen wurden unter aseptischen Bedingungen mit Gefriertrocknungsstopfen verschlossen. Die Flaschen wurden verbördelt und unter Lichtausschluß und definierten Temperaturbedingungen gelagert. Nach den entsprechenden Lagerzeiten wurden mit den in Beispiel 1 beschriebenen Methoden analytische Untersuchungen durchgeführt (vgl. Tabelle 10).

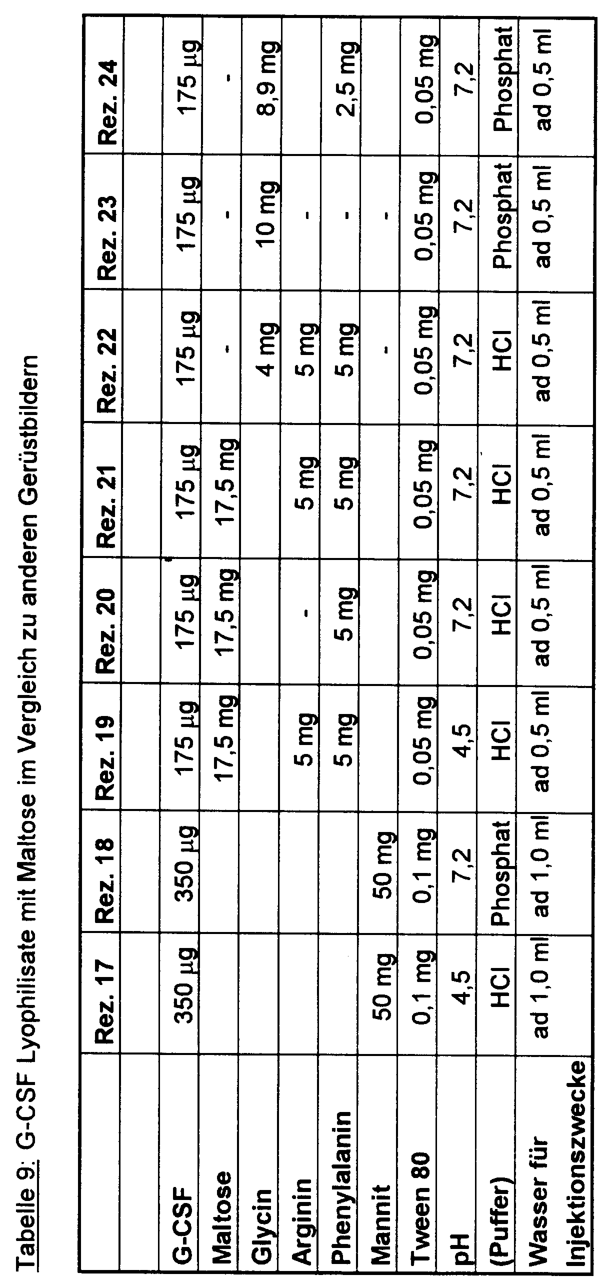

**Tabelle 10**

| sind die erhaltenen Analysendaten für die genannten Rezepturen zusammengefaßt: | | | | |
|---|---|---|---|---|
| **Rezeptur** | **Lagerung** | **4 Wochen** | | **4 Wochen Western Blot** |
| | | **RP-HPLC % G-CSF** | **SEC-HPLC % G-CSF** | |
| | | | | |
| **17** | 8 °C | > 99 | > 99 | 3,6 % Dimere |
| | 30 °C | 94 | 92 | 9,6 % Dimere |
| | 40 °C | | | 14,4 % Dimere |
| **18** | 8 °C | 69 | 60 | Aggregate |
| | 30 °C | 44 | 36 | Aggregate |
| | 40 °C | 13 | 12 | Aggregate |
| **19** | 8 °C | > 99 | > 99 | 1,0 % Dimere |
| | 30 °C | > 99 | 95,5 | 0,5 % Dimere |
| | 40 °C | > 99 | 97,5 | 0,5 % Dimere |
| **20** | 8 °C | > 99 | > 99 | 1,6 % Dimere |
| | 30 °C | > 99 | > 99 | 1,4 % Dimere |
| | 40 °C | > 99 | > 99 | 2,3 % Dimere |
| **21** | 8 °C | > 99 | > 99 | 1,5 % Dimere |
| | 30 °C | > 99 | 97,5 | 2,1 % Dimere |
| | 40 °C | > 99 | 97 | 2,0 % Dimere |
| **22** | 8 °C | > 99 | > 99 | 2,8 % Di/Aggregat |
| | 30 °C | 96 | 96 | 3,0 % Di/Aggregat |
| | 40 °C | | | 12 % Di/Aggregate |
| **23** | 8 °C | > 99 | > 99 | 6,8 % Dimere |
| | 30 °C | 91,5 | 92 | Aggregate |
| | 40 °C | 79 | 74 | Aggregate |
| **24** | 8 °C | > 99 | > 99 | 10,8 % Dimere |
| | 30 °C | 88 | 85 | Aggregate |
| | 40 °C | 67 | 60 | Aggregate |

### Beispiel 8:

### Standzeit erfindungsgemäßer wiederaufgelöster Lyophilisate mit pH 7,4

Es wurde folgende Zusammensetzung hergestellt:

| **mg/ml** | **Rezeptur 25** |
|---|---|
| | |
| **G-CSF** | 0,35 |
| **Polysorbat 80** | 0,1 |
| **Maltose** | 50 |
| **Arginin** | 10 |
| **Phenylalanin** | 10 |
| **Salzsäure** | ad pH 7,4 |

Die genannten Hilfsstoffe wurden in 1 ml Wasser für Injektionszwecke gelöst, G-CSF wurde zugefügt und der pH-Wert auf pH 7,4 eingestellt. Die Lösung wurde durch einen sterilisierten Membranfilter der Porenwerte 0,2 µm sterilfiltriert und danach in Injektionsflaschen der hydrolytischen Klasse 1 abgefüllt.

Nach dem Aufsetzen geeigneter Gefriertrocknungsstopfen wurde die Zubereitung bei einer Haupttrocknungstemperatur von - 25 °C und einer Nachtrocknungstemperatur von + 8 °C bis zu einer Restfeuchte von < 5 % gefriertrocknet. Die getrockneten Lyophilisate wurden mit Stickstoff belüftet und verschlossen.

Nach 6monatiger Lagerung bei 4 - 8 °C wurden die Lyophilisate mit 1 ml Wasser für Injektionszwecke aufgelöst und danach 24 Stunden bei Raumtemperatur stehengelassen.

Nach dieser Standzeit zeigte sich mit den in Beispiel 1 beschriebenen Untersuchungsmethoden zur biologischen Wirksamkeit (NFS-60 Test), Proteingehalt (Photometrie OD 280) und Reinheit (Western Blot), Reinheit (SEC HPLC), Reinheit (SDS Page) und Reinheit (RP HPLC) keine Veränderung gegenüber den unmittelbar nach dem Auflösen untersuchten Proben. Auch Trübungsmessungen - selbst unter mechanischer Belastung - ergaben sehr niedrige Trübungswerte.

Damit wird deutlich, daß wiederaufgelöste Lyophilisate der erfindungsgemäßen Rezeptur mit Maltose und Argininpuffer bei pH 7,4 eine für die klinische Anwendung ausreichende Standzeit besitzen.

### Beispiel 9:

### Stabilität erfindungsgemäßer Lyophilisate, die Maltose, Raffinose, Saccharose oder Trehalose enthalten, nach 13-wöchiger Lagerung bei 40 °C

Gemäß Beispiel 8, Rezeptur 25, wurden drei Lyophilisate hergestellt, die 50 mg/ml Maltose oder die gleiche Gewichtsmenge von a) Raffinose oder b) Saccarose oder c) Trehalose enthielten.

Alle Lyophilisate wurden 13 Wochen bei Temperaturen von 5 °C, 25 °C, 30 °C und 40 °C eingelagert, danach aufgelöst und visuell und mit den in Beispiel 1 beschriebenen Untersuchungsmethoden SEC HPLC, RP HPLC, Western Blot und SDS Page untersucht.

In allen Fällen ergaben sich klare farblose Lösungen. In der SEC HPLC betrug die Größe der Produktpeaks > 98 % und die der Dimeren/Aggregate < 1 %. In der RP HPLC erreichte der Produktpeak 100 %, Nebenpeaks waren nicht nachweisbar, der Hauptpeak entsprach dem Arbeitsstandard. Im SDS-Page waren keine Abbauprodukte, Dimere oder Aggregate nachweisbar.

**Tabelle**

| nach 13 Wochen Lagerzeit | | | | |
|---|---|---|---|---|
| **Temp.** | **SEC HPLC % G-CSF** | **RP HPLC % G-CSF** | **Western Blot % Aggregate** | **SDS-Page % Nebenbande** |
| | | | | |
| **5 °C** | > 98 % | 100 % | n.n. | < 1 |
| **20 °C** | > 98 % | 100 % | n.n. | < 1 |
| **30 °C** | > 98 % | 100 % | n.n. | < 1 |
| **40 °C** | > 98 % | 100 % | n.n. | < 1 |

### Beispiel 10:

### Stabilität erfindungsgemäßer Maltose-Lyophilisate mit Argininphosphat- und Argininchloridpuffern mit pH 4,5 und pH 7,2 nach 13-wöchiger Lagerung bei 30 °C

Gemäß der in Beispiel 8 beschriebenen Herstellung wurden die folgenden Rezepturen, die sich lediglich durch Puffer und pH-Wert unterscheiden, hergestellt:

| | **Rezeptur 26** | **Rezeptur 27** | **Rezeptur 28** | **Rezeptur 29** |
|---|---|---|---|---|
| | | | | |
| **G-CSF** | 0,35 mg | 0,35 mg | 0,35 mg | 0,35 mg |
| **Polysorbat 80** | 0,1 mg | 0,1 mg | 0,1 mg | 0,1 mg |
| **Phenylalanin** | 10 mg | 10 mg | 10 mg | 10 mg |
| **Arginin** | 10 mg | 10 mg | 10 mg | 10 mg |
| **Maltose** | 47,5 mg | 47,5 mg | 47,5 mg | 47,5 mg |
| **Phosphorsäure** | ad pH 4,5 | ad pH 7,2 | | |
| **Salzsäure** | | | ad pH 4,5 | ad pH 7,2 |

Die Zubereitungen wurden bei Temperaturen von 4 bis 8 °C, 20 - 25 °C sowie 30 °C eingelagert, nach 13 Wochen in 1 ml Wasser für Injektionszwecke aufgelöst und mit den in Beispiel 1 beschriebenen Untersuchungsmethoden RP HPLC, SEC HPLC (Reinheit) und Western Blot (Abbau, Dimerisation und Aggregatbildung) untersucht. Die Ergebnisse sind in Tabelle 11 dargestellt und zeigen, daß die erfindungsgemäßen Lyophilisate mit pH 4,5 und 7,2 auch nach 13-wöchiger Lagerung bei 30 °C stabil sind.

**Tabelle 11**

| Ergebnisse nach 13 Wochen 30 °C | | | | | |
|---|---|---|---|---|---|
| | **RP-HPLC** | **SEC HPLC** | **Western Blot** | | |
| | **% Nebenpeaks** | | **Abbau** | **Dimere** | **Aggregat** |
| | | | | | |
| **Rezeptur 26** | < 1 | < 1 | n.n. | n.n. | n.n. |
| **Rezepur 27** | < 1 | < 1 | n.n. | n.n. | n.n. |
| **Rezeptur 28** | < 1 | < 1 | n.n. | < 1 % | n.n. |
| **Rezeptur 29** | < 1 | < 1 | n.n. | < 1 % | n.n. |
| n.n. = nicht nachweisbar | | | | | |

### Beispiel 11:

### Stabilität erfindungsgemäßer Maltose-Lyophilisate mit Argininphosphat- und Argininchloridpuffer mit pH 7,4 nach 4- und 13-wöchiger Lagerung bei 40 °C

Es wurden in gleicher Weise wie in Beispiel 8 Rezepturen hergestellt und deren pH-Wert einmal mit Salzsäure und einmal mit Phosphorsäure auf 7,4 eingestellt:

| | **Rezeptur 30** | **Rezeptur 31** |
|---|---|---|
| | | |
| **G-CSF** | 0,35 mg | 0,35 mg |
| **Polysorbat 80** | 0,1 mg | 0,1 mg |
| **Phenylalanin** | 10 mg | 10 mg |
| **Arginin** | 10 mg | 10 mg |
| **Maltose** | 47,5 mg | 47,5 mg |
| **Phosphorsäure** | pH 7,4 | |
| **Salzsäure** | | pH 7,4 |

Diese zwei Zubereitungen wurden bei Temperaturen von 4 - 8 °C sowie 40 °C 4 und 13 Wochen eingelagert. Die Untersuchungsergebnisse (Western Blot, SDS-Page) nach 13 Wochen Lagerung sind in nachfolgender Tabelle 12 dargestellt. Die Ergebnisse nach 4-wöchiger Lagerung sind identisch.

Die Ergebnisse belegen, daß die erfindungsgemäßen Maltose-Lyophilisate mit pH 7,4 auch nach 13-wöchiger Lagerung bei 40 °C stabil sind.

**Tabelle 12**

| Ergebnisse nach 13 Wochen 40 °C | | | | | | | |
|---|---|---|---|---|---|---|---|
| | **Lager temp.** | **Western Blot, nicht reduz.** | | **SDS-Page, reduz.** | | | **Restfeuchte %** |
| | | **Aggr. < 1 %** | **Dimere < 2 %** | **Produkt-Bd.** | **Abbauprod.** | **Zusatzbd.** | |
| | | | | | | | |
| **Rezeptur 30** | 5 °C | n.n. | n.n. | < 98 % | < 1 % | n.n. | 1,4 |
| | 40 °C | n.n. | n.n. | < 98 % | < 1 % | n.n. | 2,0 |
| **Rezeptur 31** | 5 °C | n.n. | n.n. | < 98 % | < 1 % | n.n. | 1,9 |
| | 40 °C | n.n. | n.n. | < 98 % | < 1 % | n.n. | 2,1 |

### Beispiel 12:

### Stabilität erfindungsgemäßer Lyophilisate mit pH 7,4 und G-CSF-Konzentrationen von 0,5 und 1,0 mg/ml nach 13-wöchiger Lagerung bei 40° C

Gemäß der in Beispiel 8 beschriebenen Herstellung wurden die folgenden Rezepturen, die sich im G-CSF-Gehalt unterscheiden, hergestellt:

| **mg/ml** | **Rezeptur 32** | **Rezeptur 33** |
|---|---|---|
| | | |
| **G-CSF** | 0,5 mg | 1,0 mg |
| **Polysorbat 80** | 0,1 mg | 0,1 mg |
| **Phenylalanin** | 10 mg | 10 mg |
| **Arginin** | 10 mg | 10 mg |
| **Maltose** | 47,5 mg | 47,5 mg |
| **Phosphorsäure** | pH 7,4 | pH 7,4 |

Die Zubereitungen wurden bei - 20 °C, 4 - 8 °C, 20 °C - 25 °C, 30 °C und 40 °C 4 Wochen und 13 Wochen gelagert, danach in 1 ml Wasser für Injektionszwecke aufgelöst und mit den in Beispiel 1 beschriebenen Untersuchungsmethoden SEC HPLC, RP HPLC, Western Blot und SDS Page untersucht (Untersuchungsergebnisse siehe Tabelle 13).

Die Ergebnisse zeigen, daß die Lyophilisate der erfindungsgemäßen Rezepturen auch bei höherer Proteinkonzentration bis zu 1 mg/ml nach 13-wöchiger Lagerung bei 40 °C stabil sind.

### Beispiel 14 :

### Langzeitstabilität über 9 Monate

Es wurden Lyophilisate gemäß der Rezeptur 31 nach Beispiel 11 hergestellt und die Zubereitungen bei Temperaturen von - 20 °C, 5 °C, 25 °C, 30 °C und 40 °C über 9 Monate gelagert und nach 3, 6 und 9 Monaten mit allen in Beispiel 1 beschriebenen Untersuchungsmethoden untersucht.

In allen geprüften Parametern war im Laufe der Lagerzeit keine Veränderung nachweisbar. Die Zubereitung erwies sich am Ende der Lagerzeiten bei allen Temperaturen als biologisch voll wirksam, wies den vollen Proteingehalt auf und zeigte in allen Reinheitsbestimmungen Banden bzw. Peaks, die weit unter 1 % des intakten G-CSF-Moleküls lagen.

Die Ergebnisse belegen, daß die erfindungsgemäßen Lyophilisate langfristig auch bei höheren Temperaturen stabil sind und somit die im Stand der Technik beschriebenen Stabilitäten bei weitem übertreffen.

**Tabelle 14**

| Lagerung bei 30 °C | | | |
|---|---|---|---|
| | **3 Monate** | **6 Monate** | **9 Monate** |
| | | | |
| **NFS 60 Test 80 - 125 %** | entspricht | entspricht | entspricht |
| **OD 280** | 358 mg | 360 mg | 352 mg |

| **SDS Page** | | | |
|---|---|---|---|
| **Nebenbande** | < 1 % | < 1 % | < 1 % |

| **Western Blot** | | | |
|---|---|---|---|
| **% Aggr.** | n.n. | n.n. | n.n. |
| **% Dimere** | < 1 % | < 1 % | < 1 % |

| **RP HPLC** | | | |
|---|---|---|---|
| **Produktpeak** | > 99 % | > 99 % | > 99 % |

| **SEC HPLC** | | | |
|---|---|---|---|
| **Produktpeak** | > 98 % | > 98 % | > 98 % |
| **Nebenpeaks** | n.n. | n.n. | n.n. |
| **Trübungsmessung TE/F** | 0,5 | 0,5 | 0,5 |

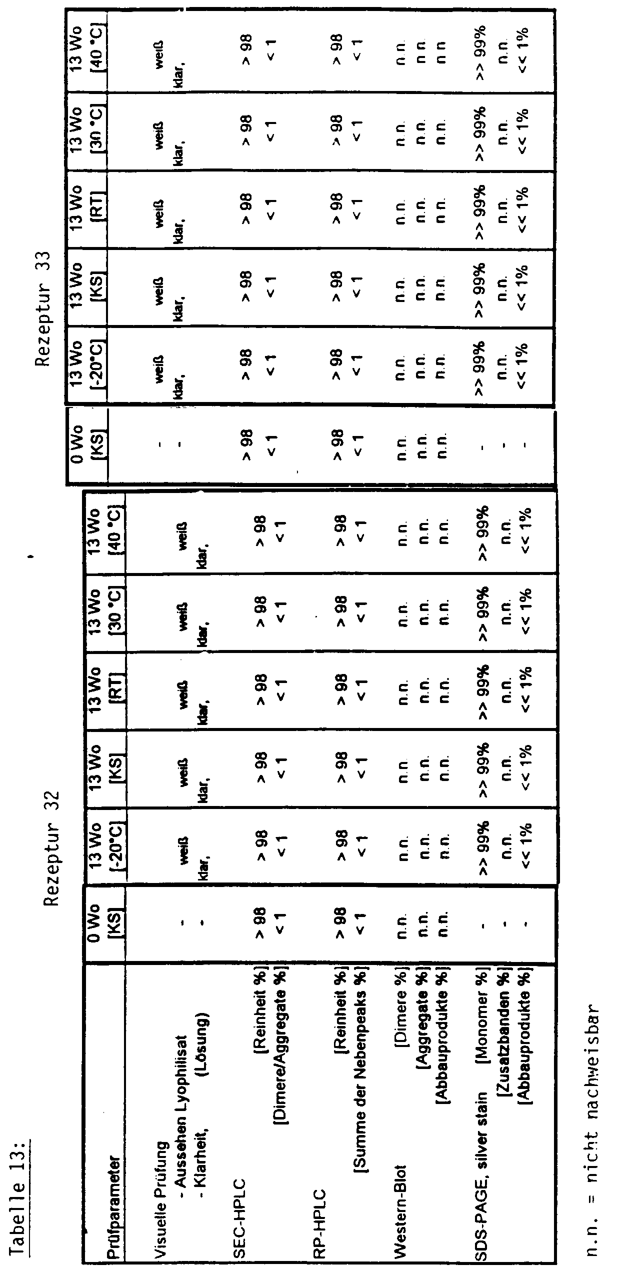

## Patentansprüche

1. Verfahren zur Herstellung von lagerstabilen, lyophilisierten pharmazeutischen Zubereitungen von G-CSF, dadurch gekennzeichnet, daß man eine wäßrige Zubereitung von G-CSF und gegebenenfalls weiteren pharmazeutischen Hilfsstoffen herstellt und die Lösung anschließend lyophilisiert, wobei zur Vermeidung eines Qualitätsverlust von G-CSF bei unkontrollierten Einfrier- und Auflauvorgängen oder bei der Lagerung bei erhöhten Temperaturen die Zusatzstoffe Maltose, Cellobiose, Gentiobiose, Isomaltose, Raffinose, Trehalose oder Aminozucker bei der Herstellung der wäßrigen Lösung zugesetzt werden.

2. Verfahren zur Herstellung von lagerstabilen, lyophilisierten pharmazeutischen Zubereitungen von G-CSF, dadurch gekennzeichnet, daß man eine wäßrige Zubereitung von G-CSF und gegebenenfalls weiteren pharmazeutischen Hilfsstoffen herstellt und die Lösung anschließend lyophilisiert, wobei zur Vermeidung des Auftretens von Aggregaten oder Dimeren von G-CSF nach Redissolution des Lyophilisates die Zusatzstoffe (1) Maltose, Raffinose, Saccharose oder Trehalose und (2) eine basische, saure oder aromatische Aminosäure bei der Herstellung der wäßrigen Lösung zugesetzt werden.

3. Verfahren nach Anspruch 1 , dadurch gekennzeichnet, daß man als Zusatzstoffe Maltose, Raffinose, Trehalose oder Aminozucker verwendet.

4. Verfahren nach Anspruch 3, dadurch gekennzeichnet, daß man als Zusatzstoffe Maltose, Raffinose, Glucosamin, Galactosamin oder Neuraminsäure verwendet.

5. Verfahren nach einem der Ansprüche 1 - 4, dadurch gekennzeichnet, daß eine physiologisch verträgliche Menge an Tensiden zugesetzt wird, die kleiner als oder maximal gleich der eingesetzten Proteinmenge an G-CSF ist.

6. Verfahren nach Anspruch 5, dadurch gekennzeichnet, daß Tenside in einer Menge von 0,5 mg/ml, vorzugsweise 0,01 - 0,1 mg/ml, zugesetzt werden.

7. Verfahren nach einem der Ansprüche 1 - 6, dadurch gekennzeichnet, daß eine physiologisch verträgliche Menge an Aminosäuren zugesetzt wird.

8. Verfahren nach Anspruch 7, dadurch gekennzeichnet, daß Arginin und/oder Phenylalanin zugesetzt wird.

9. Verfahren nach einem der Ansprüche 1 - 8, dadurch gekennzeichnet, daß als physiologisch verträgliche Hilfsstoffe Antioxidantien, Komplexbildner, Puffer, Säuren, Basen oder Isotonisierungsmittel zugesetzt werden.

10. Verfahren nach einem der Ansprüche 1 - 9, dadurch gekennzeichnet, daß als physiologisch verträglicher Hilfsstoff Phosphat- oder Acetatpuffer zugesetzt wird.

11. Verfahren nach einem der Ansprüche 1 - 10, dadurch gekennzeichnet, daß als physiologisch verträgliche Hilfsstoffe Argininphosphat, Argininchlorid oder Arginincitratpuffer, vorzugsweise mit einem pH-Wert von 7 - 8, zugesetzt wird.

12. Verwendung von Maltose, Cellobiose, Gentiobiose, Isomaltose, Raffinose, Saccharose, Trehalose, Glucosamin, Galactosamin oder Neuraminsäure zur Vermeidung eines Qualitätsverlustes von G-CSF beim Einfrieren oder bei der Lagerung bei erhöhten Temperaturen von G-CSF-enthaltenden pharmazeutischen Zubereitungen.

13. Lyophilisierte pharmazeutische Zubereitung von G-CSF enthaltend Maltose, Cellobiose, Gentiobiose, Isomaltose, Raffinose, Glucosamin, Galactosamin oder Neuraminsäure.

14. Lyophilisierte pharmazeutische Zubereitung von G-CSF- enthaltend (1) Maltose, Raffinose, Saccharose oder Trehalose und (2) eine basische, saure oder aromatische Aminosäure.

15. Lyophilisierte pharmazeutische Zubereitung von G-CSF nach Anspruch 13 oder 14 im wesentlichen frei von proteinartigen oder polymeren Hilfsstoffen.

16. Wäßrige pharmazeutische Zubereitung von G-CSF erhältlich durch Redissolution des Lyophilisates nach einem der Ansprüche 13 - 15.

17. Wäßrige pharmazeutische Zubereitung nach Anspruch 16, dadurch gekennzeichnet, daß die Lösung einen pH-Wert von 6,5 - 8 oder 3 - 5 aufweist, vorzugsweise einen pH-Wert von 7,0 - 7,5.

## Claims

1. Process for the production of lyophilized pharmaceutical preparations of G-CSF which are stable on storage, wherein an aqueous preparation of G-CSF and if desired further pharmaceutical auxiliary substances is prepared and subsequently the solution is lyophilized wherein the additives maltose, cellobiose, gentiobiose, isomaltose, raffinose, trehalose or amino sugar are added in the production of the aqueous solution in order to avoid loss of quality of G-CSF during uncontrolled freezing and thawing processes or during storage at increased temperatures.

2. Process for the production of lyophilized pharmaceutical preparations of G-CSF which are stable on storage, wherein an aqueous preparation of G-CSF and if desired further pharmaceutical auxiliary substances is prepared and subsequently the solution is lyophilized wherein the additives (1) maltose, raffinose, sucrose or trehalose and (2) a basic, acidic or aromatic amino acid are added in the production of the aqueous solution in order to avoid the occurrence of aggregates or dimers of G-CSF after redissolution of the lyophilisate.

3. Process as claimed in claim 1, wherein maltose, raffinose, trehalose or amino sugar are used as additives.

4. Process as claimed in claim 3, wherein maltose, raffinose, glucosamine, galactosamine or neuraminic acid are used as additives.

5. Process as claimed in one of the claims 1 - 4, wherein a physiologically tolerated amount of surfactants is added which is smaller than or at most the same as the amount of G-CSF protein used.

6. Process as claimed in claim 5, wherein surfactants are added in an amount of 0.5 mg/ml, preferably of 0.01 - 0.1 mg/ml.

7. Process as claimed in one of the claims 1 - 6, wherein a physiologically tolerated amount of amino acids is added.

8. Process as claimed in claim 7, wherein arginine and/or phenylalanine is added.

9. Process as claimed in one of the claims 1 - 8, wherein antioxidants, complexing agents, buffers, acids, bases or isotonizing agents are added as the physiologically tolerated auxiliary substances.

10. Process as claimed in one of the claims 1 - 9, wherein phosphate buffer or acetate buffer is added as the physiologically tolerated auxiliary substance.

11. Process as claimed in one of the claims 1 - 10, wherein arginine phosphate buffer, arginine chloride buffer or arginine citrate buffer preferably with a pH value of 7 - 8 is added as a physiologically tolerated auxiliary substance.

12. Use of maltose, cellobiose, gentiobiose, isomaltose, raffinose, sucrose, trehalose, glucosamine, galactosamine or neuraminic acid to avoid loss of G-CSF quality during freezing or during storage at increased temperatures of pharmaceutical preparations containing G-CSF.

13. Lyophilized pharmaceutical preparation of G-CSF containing maltose, cellobiose, gentiobiose, isomaltose, raffinose, glucosamine, galactosamine or neuraminic acid.

14. Lyophilized pharmaceutical preparation of G-CSF containing (1) maltose, raffinose, sucrose or trehalose and (2) a basic, acidic or aromatic amino acid.

15. Lyophilized pharmaceutical preparation of G-CSF as claimed in claim 13 or 14 that is essentially free of protein-like or polymeric auxiliary substances.

16. Aqueous pharmaceutical preparation of G-CSF obtainable by redissolution of the lyophilisate as claimed in one of the claims 13 - 15.

17. Aqueous pharmaceutical preparation as claimed in claim 16, wherein the solution has a pH value of 6.5 - 8 or 3 - 5, preferably a pH value of 7.0 - 7.5.

## Revendications

1. Procédé d'obtention de préparations pharmaceutiques lyophilisées de G-CSF (facteur stimulant les colonies de granulocytes) stables au stockage, caractérisé par le fait que l'on prépare une préparation aqueuse à base de G-CSF et éventuellement d'adjuvants pharmaceutiques et ensuite, on lyophilise la solution, procédé dans lequel, pour empêcher une baisse de qualité du G-CSF provoquée par des processus de congélation-décongélation incontrôlés, ou par stockage à température élevée, on ajoute à la solution aqueuse, lors de sa préparation, les adjuvants que sont le maltose, le cellobiose, le gentiobiose, l'isomaltose, le raffinose, le tréhalose ou le sucre aminé.

2. Procédé d'obtention de préparations pharmaceutiques lyophilisées de G-CSF stables au stockage, caractérisé par le fait que l'on prépare une préparation aqueuse à base de G-CSF et éventuellement d'adjuvants pharmaceutiques et ensuite, on lyophilise la solution, procédé dans lequel, pour éviter l'apparition d'agrégats ou de dimères de G-CSF, après redissolution du lyophilisat, on ajoute à la solution aqueuse, lors de sa préparation, les adjuvants que sont (1) le maltose, le raffinose, le saccharose ou le tréhalose, et (2) un acide aminé basique, acide ou aromatique.

3. Procédé selon la revendication 1, caractérisé par le fait que, comme adjuvant, on utilise du maltose, du raffinose, du tréhalose ou du sucre aminé.

4. Procédé selon la revendication 3, caractérisé par le fait que, comme adjuvant, on utilise du maltose, du raffinose, de la glucosamine, de la galactosamine ou de l'acide neuraminique.

5. Procédé selon l'une quelconque des revendications 1-4, caractérisé par le fait que l'on ajoute un tensioactif en une quantité physiologiquement acceptable qui est inférieure ou au maximum égale à la quantité de protéine mise en oeuvre en tant que G-CSF.

6. Procédé selon la revendication 5, caractérisé par le fait que le tensioactif est ajouté en une quantité de 0,5 mg/ml, de préférence de 0,01-0,1 mg/ml.

7. Procédé selon l'une quelconque des revendications 1-6, caractérisé par le fait que l'on ajoute une quantité physiologiquement acceptable d'acide aminé.

8. Procédé selon la revendication 7, caractérisé par le fait que l'on ajoute de l'arginine et/ou de la phénylalanine.

9. Procédé selon l'une quelconque des revendications 1-8, caractérisé par le fait que, comme adjuvant physiologiquement acceptable, on ajoute des antioxydants, des complexants, des tampons, des acides, des bases ou des agents d'isotonicité.

10. Procédé selon l'une quelconque des revendications 1-9, caractérisé par le fait que, comme adjuvant physiologiquement acceptable, on ajoute du tampon phosphate ou acétate.

11. Procédé selon l'une quelconque des revendications 1-10, caractérisé par le fait que, comme adjuvant physiologiquement acceptable, on ajoute du tampon phosphate d'arginine, chlorure d'arginine ou citrate d'arginine, ayant de préférence un pH de 7-8.

12. Utilisation de maltose, cellobiose, gentiobiose, isomaltose, raffinose, saccharose, tréhalose, glucosamine, galactosamine, ou acide neuraminique pour éviter la baisse de qualité du G-CSF, provoquée par congélation ou par stockage à température, élevée de préparations pharmaceutiques contenant du G-CSF.

13. Préparation pharmaceutique lyophilisée de G-CSF contenant du maltose, du cellobiose, du gentibiose, de l'isomaltose, du raffinose, de la glucosamine, de la galactosamine ou de l'acide neuraminique.

14. Préparation pharmaceutique lyophilisée de G-CSF contenant (1) du maltose, du raffinose, du saccharose ou du tréhalose, et (2) un acide aminé basique, acide ou aromatique.

15. Préparation pharmaceutique lyophilisée de G-CSF selon la revendication 13 ou 14, essentiellement dépourvue d'adjuvants de type protéiné ou polymères.

16. Préparation pharmaceutique aqueuse de G-CSF, pouvant être obtenue par redissolution du lyophilisat selon l'une des revendications 13-15.

17. Préparation pharmaceutique aqueuse de G-CSF selon la revendication 16, caractérisée par le fait que la solution présente un pH de 6,5-8 ou de 3-5, de préférence un pH de 7,0-7,5.
